(19) Europäisches Patentamt / European Patent Office / Office européen des brevets

(11) **EP 3 652 531 B1**

(12) **FASCICULE DE BREVET EUROPEEN**

(45) Date de publication et mention
de la délivrance du brevet:
**11.12.2024 Bulletin 2024/50**

(21) Numéro de dépôt: **18752568.8**

(22) Date de dépôt: **09.07.2018**

(51) Classification Internationale des Brevets (IPC):
**G01N 33/18** (2006.01)  **G01N 21/31** (2006.01)
**G01N 21/35** (2014.01)  **G01N 21/39** (2006.01)

(52) Classification Coopérative des Brevets (CPC):
**G01N 33/18; B01D 19/0005; G01N 30/00;**
G01N 21/35; G01N 2021/391

(86) Numéro de dépôt international:
**PCT/FR2018/051715**

(87) Numéro de publication internationale:
**WO 2019/012212 (17.01.2019 Gazette 2019/03)**

(54) **DISPOSITIF ET MÉTHODE D'ANALYSE EN CONTINU DE LA CONCENTRATION DU CARBONE INORGANIQUE DISSOUS (DIC) ET DE SES COMPOSITIONS ISOTOPIQUES EN CARBONE ET OXYGÈNE**

VORRICHTUNG UND VERFAHREN ZUR KONTINUIERLICHEN ANALYSE DER KONZENTRATION VON GELÖSTEM ANORGANISCHEM KOHLENSTOFF (DIC) UND DER ISOTOPENKOHLE UND DEREN SAUERSTOFFZUSAMMENSETZUNGEN

DEVICE AND METHOD FOR CONTINUOUS ANALYSIS OF THE CONCENTRATION OF DISSOLVED INORGANIC CARBON (DIC) AND OF THE ISOTOPIC CARBON AND OXYGEN COMPOSITIONS THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.07.2017 FR 1756500**

(43) Date de publication de la demande:
**20.05.2020 Bulletin 2020/21**

(73) Titulaires:
• **Centre National de la Recherche Scientifique**
**75016 Paris (FR)**
• **Université De Bretagne Occidentale - UBO**
**29200 Brest (FR)**

(72) Inventeurs:
• **SANS-JOFRE, Pierre**
**94220 Charenton-le-Pont (FR)**
• **LALONDE, Stefan**
**29290 Tréouergat (FR)**
• **LIORZOU, Céline**
**29200 Brest (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
WO-A1-2015/179871    CN-A- 105 806 689
CN-U- 201 555 755    US-A- 4 277 438
US-A- 5 459 075    US-A1- 2006 210 961
US-A1- 2010 212 406    US-B2- 8 114 676

EP 3 652 531 B1

**Description**

**Domaine technique de l'invention**

La présente invention concerne un nouveau dispositif et une nouvelle méthode d'analyse pour obtenir la concentration du carbone inorganique dissous (DIC) et sa concentration isotopique en carbone et oxygène, en continu.

La présente invention trouve des applications dans différents domaines comme par exemple : la surveillance des émissions de polluants dans les rivières ; la surveillance de la qualité des eaux (nappe phréatique, piscine, station de traitement des eaux usées, industrie pharmaceutique, production de matériaux électroniques, etc...) ; la détection de fraude des vins et champagnes par analyse isotopique, l'observation volcanique ; la biologie, la détection des blooms planctoniques et eutrophisation des lacs ; le suivi en continu de l'acidification des océans dû au réchauffement climatique ; le suivi et la contamination des nappes phréatiques. La présente invention donne également accès à un nouveau champ disciplinaire de recherche que ce soit en biologie ou géologie, qu'est l'analyse des variations rapides isotopiques et en concentration du DIC sur des périodes de plus ou moins longue durée.

Dans la description ci-dessous, les références entre crochets (**[ ]**) renvoient à la liste des références présentée à la fin du texte.

**État de la technique**

La méthode la plus commune pour l'analyse de la concentration du DIC est la titration d'un échantillon liquide avec une solution basique de concentration connue, soit le $Na_2CO_3$, soit le NaOH, au point d'équivalence de l'indicateur phénolphtaléine (pH 8,3) (e.g., Method 4500-$CO_2$ dans Rice EW, Bridgewater L, Association APH, Association AWW, Fédération WE « Standard Methods for the Examination of Water and Wastewater », Am. Public Health Assn., 2012) [1]. Il existe d'autres systèmes qui réalisent une conversion du DIC en $CO_2$ via l'acidification d'un échantillon liquide dans une chambre de réaction suivi par le passage du $CO_2$ produit vers un système de détection. Ces systèmes de détection sont basés principalement sur des techniques d'absorption laser infra-rouge (e.g., AS-C3, Apollo SciTech, LLC), mais aussi sur d'autres techniques de détection comme des changements colorimétriques (e.g., CM140 Total Inorganic Carbon Analyzer, UIC, Inc.). Toutefois l'ensemble de ces méthodes restent des méthodes d'analyse en DIC ponctuelles.

La méthode utilisée jusqu'à présent pour l'analyse de la composition isotopique en oxygène et en carbone du DIC repose sur un mélange ponctuel de l'échantillon aqueux avec de l'acide orthophosphorique dans un tube fermé hermétiquement et vidé de son $CO_2$ initial (Assayag et al., 2006) [2]. Ce mélange peut aussi être effectué dans une chambre de réaction (Bass et al., 2012) [3]. Cette étape est suivie d'un temps d'équilibration allant d'une dizaine de minutes et pouvant atteindre plusieurs heures (Bass et al., 2012 ; Assayag et al., 2006). Le $CO_2$ produit est alors envoyé vers un système de détection comprenant principalement des spectromètres de masse en phase gazeuse (IRMS, e.g., Delta V, Thermo Fisher) et plus rarement des spectromètres infra-rouge (e.g., Delta Ray, Picarro). Ces deux méthodes sont des techniques d'analyses ponctuelles qui nécessitent des prélèvements d'échantillons qui sont stockés puis traités par la suite en laboratoire. Les techniques utilisées sont chronophages et coûteuses, et ne permettent pas d'effectuer des analyses en continu au laboratoire ou même sur le terrain.

Il est décrit dans l'art quelques systèmes de mesure en continu de la concentration du DIC (Demande de Brevet US 2010/0212406) [4]. Toutefois, ces techniques présentent généralement une rapidité de mesure insuffisante (e.g., supérieure aux dizaines de secondes), et ne sont pas compatibles avec un objectif d'obtention des rapports isotopiques.

Le brevet US 8,114,676 [7] décrit un dispositif et procédé d'analyse de DIC dans un échantillon aqueux qui ne comprend pas la mesure isotopique en carbone et oxygène.

Il existe donc un besoin pour une méthode d'analyse continue de la concentration et de la composition isotopique en carbone et oxygène du carbone inorganique dissous (DIC).

**Description de l'invention**

Les Inventeurs ont mis au point un système permettant l'analyse en concentration et l'analyse des compositions isotopiques en carbone et en oxygène du Carbone Inorganique Dissous (DIC), en continu, et à l'échelle de la seconde, via la production chimique permanente de $CO_2$ (gaz) à partir du DIC, puis une séparation gaz-liquide amenant le flux continu de $CO_2$ produit vers un détecteur de type spectromètre laser infra-rouge. Une pompe péristaltique contrôle le mélange des deux liquides (échantillon et acide anhydride) qui entraine une production de $CO_2$. Ce $CO_2$ est séparé des liquides résiduels et acheminé grâce à un gaz vecteur vers un analyseur. Ce système permet donc le suivi en continu des concentrations et compositions isotopiques en carbone et en oxygène du DIC. Les tests ont montré que les gaz récupérés permettent d'obtenir la concentration en DIC, ainsi que ses compositions isotopiques précises en carbone et en oxygène, en continu, sans stagnation dans une chambre fermée momentanément.

Les inventeurs ont ainsi réalisé pour la première fois une production de $CO_2$ constante dans le temps, grâce à un rapide dégazage du $CO_2$ à partir d'un réservoir liquide, pour une concentration donnée de $CO_2$. Ceci a permis d'établir un rapport linéaire entre la concentration du $CO_2$ du flux sortant du séparateur gaz-liquide et la concentration du DIC de l'échantillon analysé. Le fait de réaliser le dégazage et la séparation rapide et efficace gaz-liquide à l'échelle de la seconde, permet de contrôler et de s'affranchir des problèmes d'équilibrage isotopique, notamment entre l'oxygène du $CO_2$ produit et celui de l'eau liquide. Ainsi, cette technique a également permis d'obtenir directement à l'échelle de la seconde, la composition isotopique en carbone et en oxygène du DIC dans l'échantillon à partir du $CO_2$ produit.

Pour arriver à une telle séparation, les Inventeurs se sont tournés vers des systèmes existants permettant la production et la séparation de gaz à partir d'un mélange de réactif et effluent à analyser : les générateurs d'hydrures. Le système d'introduction, commercialisé par une poignée d'industriels (*e.g.*, Teledyne-CETAC, ESI Inc., etc...), sert à mélanger un réactif très réducteur (*e.g.*, NaBH4) avec de l'acide (HCl) et un effluent dans le but de générer des hydrures gazeux de métaux et métalloïdes (*e.g.*, Ge, Se, Sb, Hg). Ces hydrures sont ensuite envoyés dans un analyseur de type ICP-AES ou ICP-MS par couplage direct via l'utilisation d'un gaz vecteur inerte (argon). Dans le cadre de la présente invention, les Inventeurs ont utilisé la haute capacité des générateurs d'hydrures à effectuer le mélange physique, la réaction chimique, la séparation gazeuse, et le prélèvement de gaz, pour l'étude des gaz naturellement dissous dans des fluides tels que le $CO_2$, mais en les détournant de leur fonctionnement initial. Pour ce faire, ils ont dû modifier le branchement d'un générateur d'hydrure (hybrideICP, modèle HG-MP2-4-A, ESI Inc.) pour produire un fluide continu de $CO_2$ de concentration proportionnelle à celle du DIC et portant sa signature isotopique (carbone et oxygène) de la manière suivante :

- Substitution des acides classiques par de l'acide orthophosphorique concentré (85-102%). Ceci a permis de diminuer l'interaction avec l'eau naturellement contenue dans les autres acides minéraux classiques (HCl, $HNO_3$, $H_2SO_4$, HF). En outre, ce nouveau montage s'est montré également efficace dans la production de $CO_2$ avec d'autres acides.
- Contrairement aux méthodes de génération d'hydrures classiques qui nécessitent un réactif réducteur pour réduire l'analyte en question vers sa forme hydrure, dans le cadre de la présente invention, aucun autre réactif n'est nécessaire pour générer du gaz (*i.e.,* du $CO_2$) à partir de l'effluent. Pour ce faire, l'entrée de réducteur de l'hybrideICP a été bouchée, ne permettant ainsi qu'une alimentation du générateur d'hydrure par deux réactifs et non plus trois : le réactif acidifiant et l'échantillon à analyser.
- Au lieu d'un vecteur de gaz inerte adapté à l'analyse spectrométrique à source de plasma (argon), des gaz compatibles avec les spectromètres dédiés à l'analyse des isotopes du carbone et de l'oxygène ont été sélectionnés. Pour les spectromètres à ratio isotopique (IRMS), l'hélium est le gaz vecteur, et pour les spectromètres de type cavity ring-down (CRDS; modèle G2131-*i*, Picarro Inc.) ou spectromètre infra-rouge à rapports isotopiques (IRIS), comme le modèle Delta ray, Thermo Fisher Inc.), il s'agit d'air synthétique sans $CO_2$. Ces choix de gaz vecteur protègent la source d'ionisation (dans le cas des IRMS) ou optimise les conditions optiques internes aux instruments (dans les types CRDS et IRIS). La présente invention s'applique également aux instruments IRMS et CRDS/IRIS dans sa capacité à fournir un flux continu de $CO_2$ portant la composition du DIC dans l'effluent rentrant dans le séparateur gaz-liquide.
- Les conditions de mélange (longueur d'une boucle de réaction entre l'acide et l'effluent, la proportion d'acide et de l'effluent dans le mélange) ainsi que la production de $CO_2$ (vitesse de la pompe, diamètre des tuyaux amenant l'acide et l'effluent dans le mélange) sont optimisés pour une production maximale de $CO_2$.
- Une sortie de gaz en excès de type « open-split » peut être ajoutée entre le séparateur gaz-liquide et le système d'analyse pour ajuster et optimiser la pression des gaz rentrant dans le système d'analyse.
- L'effluent est aspiré, mélangé et subi une séparation gaz-liquide. Le $CO_2$ évolué est apporté en flux continu au système d'analyse, sur des échelles de temps extrêmement courtes (généralement à l'échelle de la seconde à la minute).
- Indépendamment du délai d'entrée de l'effluent dans le système séparateur gaz-liquide et l'arrivée du $CO_2$ produit vers le système d'analyse, la présente invention permet l'analyse de l'évolution des concentrations et compositions isotopiques du DIC à une échelle qui n'est limitée que par le temps de résidence des gaz dans le séparateur gaz-liquide et des processus de mélange lors du transport entre le séparateur gaz-liquide et l'analyseur. Selon un premier prototype (volume du séparateur gaz-liquide d'environ 10 $cm^3$), l'échelle a été estimée de l'ordre de 1 à 6 secondes dépendant principalement du volume de la chambre de séparation. Cette échelle de temps peut encore être diminuée en-deçà de la seconde en réduisant le volume du séparateur gaz-liquide, en augmentant le flux continu du gaz vecteur et en raccourcissant la distance des connexions entre le séparateur gaz-liquide et l'analyseur.

Ainsi le montage de la présente invention a permis d'obtenir directement et à l'échelle de la seconde, la concentration et les compositions isotopiques en carbone et en oxygène du DIC dans des effluents liquides.

La présente invention concerne donc une méthode et un dispositif d'analyse de la concentration et des compositions isotopiques en carbone et en oxygène du Carbone Inorganique Dissous (DIC) dans un échantillon liquide.

Ledit dispositif comprend ou consiste en un assemblage de tubes connectés à une pompe péristaltique (5) qui permet l'injection et le mélange de deux fluides avec un flux précis. Le mélange consiste en un échantillon liquide et un réactif acidifiant qui sert à forcer l'équilibre dans les réactions thermodynamiques du système DIC vers le $CO_2$ gazeux. Ce mélange est libéré dans une enceinte en verre, chambre de séparation gaz-liquide (12), qui sert à récupérer le gaz produit dans un flux continu de gaz vecteur (16), et d'envoyer le mélange de gaz contenant le $CO_2$ issue du DIC dans un analyseur de type spectromètre laser infra-rouge (19). Ces tubes sont connectés chacun à un réservoir. Le réservoir (1) contient l'échantillon liquide à analyser. Le réservoir (2) contient un acide anhydrique (e.g., $H_3PO_4$>85%). Ces deux fluides sont acheminés vers la pompe péristaltique (5) avec des flux constants, F1 (3) et F2 (4), respectivement. Ces fluides sont mélangés dynamiquement dans une chambre de mélange (6) puis véhiculés dans une boucle réactionnelle (7) dont la longueur peut varier en fonction du type d'échantillon à analyser. Dans cette boucle réactionnelle (7), une réaction acide-base permet de convertir le DIC en $CO_2$ gazeux, en présence d'un excès d'acide. Ce mélange de gaz et liquide est acheminé dans une chambre de séparation (12) qui permet de récupérer le $CO_2$ gazeux produit. Cette chambre (12) est connectée par le bas à une bouteille de gaz vecteur (8) contenant de l'air synthétique sans $CO_2$. Ce gaz est généralement constitué de 20% de dioxygène, 79% de diazote et de 1% d'argon. Ce mélange peut varier en fonction du système de détection utilisé. Le flux (11) est contrôlé par un détendeur (9) et un boitier de contrôle (10) qui permet de modifier, par ordinateur, ce flux (11), afin d'optimiser la quantité de $CO_2$ arrivant au système de détection (19). Le gaz vecteur (sans $CO_2$) est envoyé dans la chambre de séparation (12) et capte le $CO_2$ produit durant la réaction dans la boucle réactionnelle (7) et libéré dans la chambre de séparation (12). Cette chambre (12) peut posséder une paroi à surface étendue et traitée (e.g., verre dépoli) de manière à optimiser le dégazage (13). D'un côté, le fluide résiduel (contenant l'acide et l'échantillon dégazé) est pompé et récupéré dans un réservoir de déchets (15), de l'autre, le gaz vecteur (16) permet de transporter le $CO_2$ dégazé de l'échantillon. Ce gaz (16) peut éventuellement passer via un open-split (17) pour un ajustement des flux en fonction des besoins du système de détection avant de passer à travers un piège à eau et à particules (18) et un analyseur de type spectromètre laser infra-rouge (19).

La présente invention a donc pour objet un dispositif d'analyse en continu de la concentration du Carbone Inorganique Dissous (DIC) et de ses compositions isotopiques en carbone et en oxygène dans un échantillon liquide, ledit dispositif comprenant ou consistant en: - une pluralité de réservoirs, chacune comprenant un fluide;

- un assemblage de tubes chacun connecté à un réservoir (1),(2) contenant chacun un fluide, et connectés à une pompe (5) positionnée en aval desdits tubes ;
- une chambre de mélange (6) positionnée en aval de la pompe (5) et dans laquelle les fluides acheminés vers la pompe (5) sont mélangés dynamiquement ;
- une boucle réactionnelle (7) positionnée en aval de la chambre de mélange (6), ladite boucle réactionnelle (7) permettant de convertir le DIC en $CO_2$ gazeux selon une réaction acide-base ;
- une chambre de séparation (12) positionnée en aval de la boucle réactionnelle (7), ladite chambre de séparation (12) étant également connectée à une bouteille de gaz vecteur (8) contenant du gaz vecteur sans $CO_2$ ;
- un détendeur (9) et un boitier de contrôle (10) positionnés entre la bouteille de gaz vecteur (8) et la chambre de séparation (12) et qui contrôlent le flux (11) de gaz vecteur envoyé de la bouteille de gaz vecteur (8) dans la chambre de séparation (12) pour capter le $CO_2$ produit dans la boucle réactionnelle (7) ;
- un réservoir de déchet (15) positionné en aval de la chambre de séparation (12) pour récupérer via la pompe (5) les fluides résiduels dégazés ;
- un piège à eau et à particules (18) positionné en aval de la chambre de séparation ;
- éventuellement une sortie de gaz en excès de type open-split (17) positionnée entre la chambre de séparation (12) et le piège à eau et à particules (17) afin d'ajuster les flux ;
- un système de détection (19) positionné en aval du piège à eau et à particules (18).

Selon un mode de réalisation particulier du dispositif de la présente invention, la chambre de réaction (12) comprend une paroi à surface étendue et traitée pour optimiser le dégazage (13). Par exemple, la paroi de la chambre de séparation (12) est en verre dépoli.

Selon un mode de réalisation particulier du dispositif de la présente invention, le réservoir (1) contient l'échantillon liquide à analyser, et le réservoir (2) contient un réactif acidifiant, de préférence un acide anhydrique, tout préférentiellement un acide orthophosphorique.

Selon un mode de réalisation particulier du dispositif de la présente invention, le système de détection (19) est un analyseur de type spectromètre infra-rouge à rapports isotopiques (IRIS).

Selon un mode de réalisation particulier du dispositif de la présente invention, le gaz vecteur est choisi parmi l'hélium et de l'air synthétique sans $CO_2$ (*i.e.* un mélange comprenant 20% oxygène, 79% azote et 1% argon).

La présente invention a également pour objet une méthode d'analyse en continu de la concentration du Carbone Inorganique Dissous (DIC) et de ses compositions isotopiques en carbone et en oxygène dans un échantillon liquide, ledit procédé comprenant ou consistant en :

a) l'acheminement de l'échantillon liquide à analyser et d'un réactif acidifiant vers une pompe (5) avec des flux constants F1 (3) et F2 (4), respectivement ;

b) l'injection et le mélange des deux fluides dans une chambre de mélange (6) ;

c) l'acheminement du mélange de fluides obtenu à l'étape b) vers une boucle réactionnelle (7) dans laquelle une réaction acide-base permet de convertir en tout ou partie le DIC en $CO_2$ gazeux ;

d) l'acheminement du mélange de gaz et de liquide obtenu à l'étape c) dans une chambre de séparation (12) ;

e) l'injection d'un flux (11) de gaz vecteur dépourvu de $CO_2$ dans la chambre de séparation (12) afin de capter le $CO_2$ gazeux produit lors de l'étape c) et libéré (13) dans la chambre de séparation (12) ;

f) l'acheminement du flux (16) de gaz vecteur contenant le $CO_2$ dégazé de l'échantillon obtenu à l'étape e) à travers un piège à eau et à particules (18), éventuellement après un passage dans un open-split (17) pour un ajustement de flux ;

g) l'acheminement du fluide résiduel composé du réactif acidifiant et de l'échantillon dégazé dans un réservoir de déchets (15) ;

h) l'acheminement du flux (16) de gaz vecteur contenant le $CO_2$ gazeux issu du DIC dans un système de détection (19) ;

i) la détermination de la concentration du Carbone Inorganique Dissous (DIC) et/ou de ses compositions isotopiques en carbone et en oxygène par ledit système de détection (19).

Selon un mode de réalisation particulier du procédé de la présente invention, l'étape a) est réalisée sous des flux constants F1 (3) d'échantillon et F2 (4) de réactif acidifiant de l'ordre d'environ 750pl/min et d'environ 190 $\mu$l/min, respectivement. Cela fait un ratio échantillon/réactif acidifiant d'environ 4.

Selon un mode de réalisation particulier du procédé de la présente invention, l'étape c) est réalisée en présence d'un excès d'acide pour une conversion optimale du DIC en $CO_2$ gazeux.

Selon un mode de réalisation particulier du procédé de la présente invention, l'étape e) est réalisée avec une boucle réactionnelle dont la longueur varie, par exemple de 0,5 à 4,5 ml, en fonction de l'échantillon à analyser.

Selon un mode de réalisation particulier du procédé de la présente invention, l'étape e) utilise un gaz vecteur choisi parmi l'hélium et l'air synthétique sans $CO_2$ (*i.e.* un mélange comprenant 20% oxygène, 79% azote et 1% argon).

Selon un mode de réalisation particulier du procédé de la présente invention, le flux (11) est contrôlé par un détendeur (9) et un boîtier de contrôle (10) afin d'optimiser la quantité de $CO_2$ gazeux arrivant au système de détection (19).

Selon un mode de réalisation particulier du procédé de la présente invention, l'étape a) est réalisée avec un réactif acidifiant qui est un acide anhydrique, de préférence un acide orthophosphorique.

Selon un mode de réalisation particulier du procédé de la présente invention, l'étape i) est réalisée à l'aide d'un analyseur de type spectromètre infra-rouge à rapports isotopiques (IRIS).

## Brève description des figures

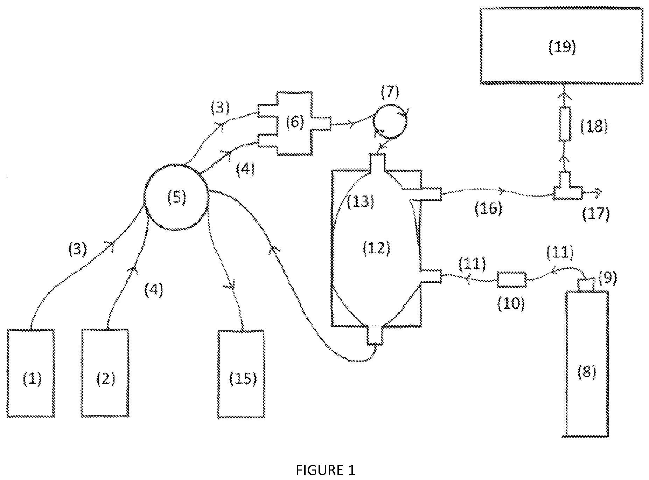

- La figure 1 représente un schéma simplifié d'un mode de réalisation d'une version exemplaire d'un dispositif de la présente invention.

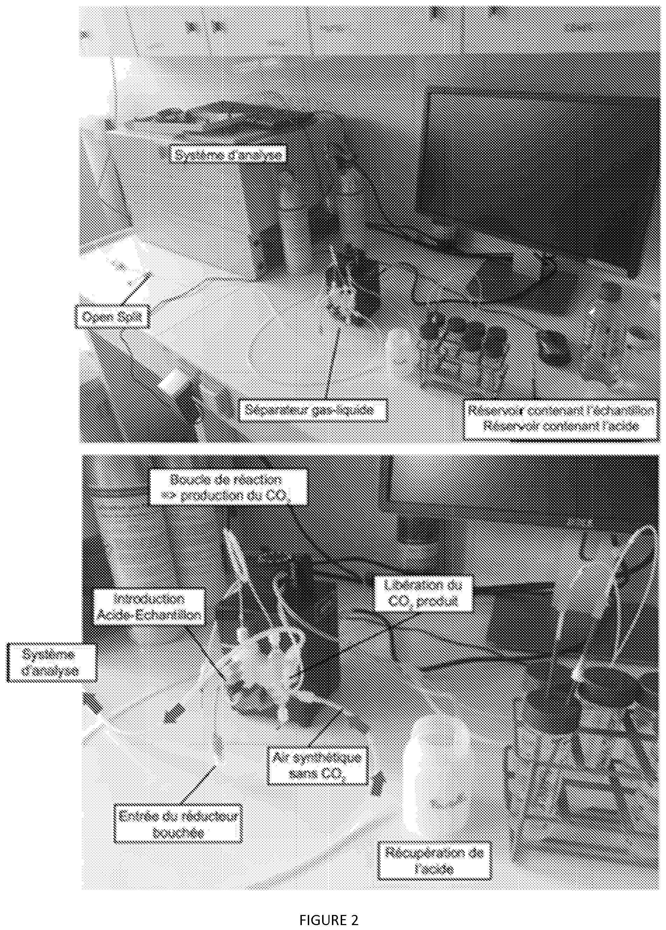

- La figure 2 représente un prototype exemplaire d'un dispositif de la présente invention.
- La figure 3 représente les résultats de mesure des rapports isotopiques en carbone (D13C) et en oxygène (D18O) du DIC de divers échantillons liquides obtenus par une méthode classique d'analyse avec équilibration et la méthode d'analyse selon la présente l'invention. (**A**) graphique de comparaison des résultats obtenus pour les deux méthodes (méthode classique et méthode en continue) pour les isotopes du carbone. (**B**) graphique de comparaison des résultats obtenus pour les deux méthodes (méthode classique et méthode en continue) pour les isotopes de l'oxygène.
- La figure 4 représente les effets de la concentration sur les valeurs isotopiques en carbone (D13C) et en oxygène (D18O) du DIC de différents échantillons liquides : (**A**) dans $Na_2CO_3$ (**B**) dans $NaHCO_3$.
- La figure 5 représente les effets de la vitesse de la pompe sur les concentrations en $CO_2$ du DIC (**A**) dans l'eau minérale Plancoët, $Na_2CO_3$ et NaHCOs, et les valeurs isotopiques en carbone du DIC (**B**) dans l'eau minérale Plancoët, $Na_2CO_3$ et $NaHCO_3$, et en oxygène du DIC (**C**) dans l'eau minérale Plancoët, $Na_2CO_3$ et $NaHCO_3$.
- La figure 6 représente les effets de la longueur de la boucle réactionnelle ou de mélange sur les concentrations en $CO_2$ du DIC (**A**) dans l'eau minérale Plancoët, $Na_2CO_3$ et $NaHCO_3$, et les valeurs isotopiques en carbone du DIC (**B**) dans l'eau minérale Plancoët, $Na_2CO_3$ et $NaHCO_3$ et en oxygène du DIC (**C**) dans l'eau minérale Plancoët, $Na_2CO_3$ et $NaHCO_3$.

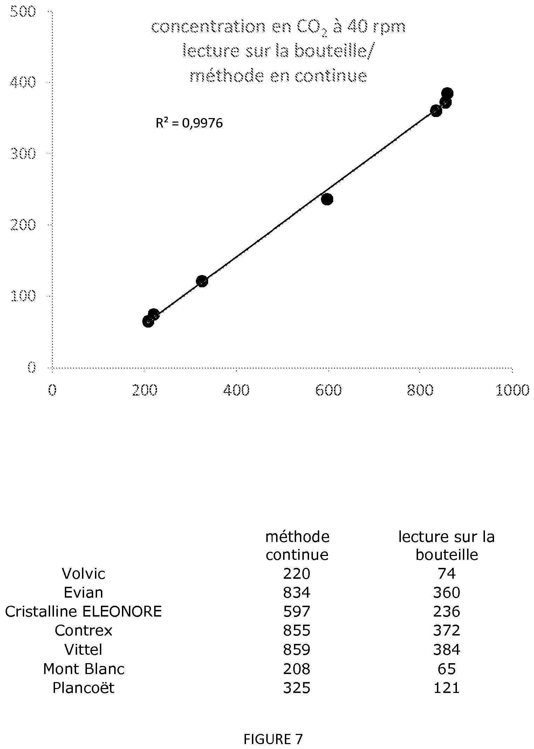

- La figure 7 présente un graphique montrant une relation linéaire entre les concentrations en DIC lue sur des bouteilles d'eau minérales et les concentrations déduites de nos analyses.

**EXEMPLES**

**EXEMPLE 1 : COMPARAISON DES COMPOSITIONS ISOTOPIQUES EN CARBONE ET EN OXYGÈNE DU DIC D'ECHANTILLONS LIQUIDES OBTENUES PAR UNE MÉTHODE CLASSIQUE D'ANALYSE AVEC ÉQUILIBRATION ET PAR LA MÉTHODE D'ANALYSE DE L'INVENTION**

Pour cette expérience, 7 eaux minérales et une eau de mer ont été mesurées en $\delta^{13}C$ et en $\delta^{18}O$ afin de comparer les mesures obtenues avec le générateur d'hydrure utilisé selon la présente invention et avec la méthode classique d'équilibration (Smajgl et al. 2017 19th EGU General Assembly, EGU2017, proceedings from the conférence held 23-28 April, 2017 in Vienna, Austria., p.9793) [6]. Les 2 types de mesures ont été effectués sur un Delta Ray. Les droites de corrélation entre les méthodes ont des coefficients de corrélation de 0,99 ;
Pour cette expérience les paramètres par défaut sont les suivants :

- vitesse de la pompe : 40 rpm
- taille de la boucle : 0,5 ml
- flux de gaz : 80 ml/min

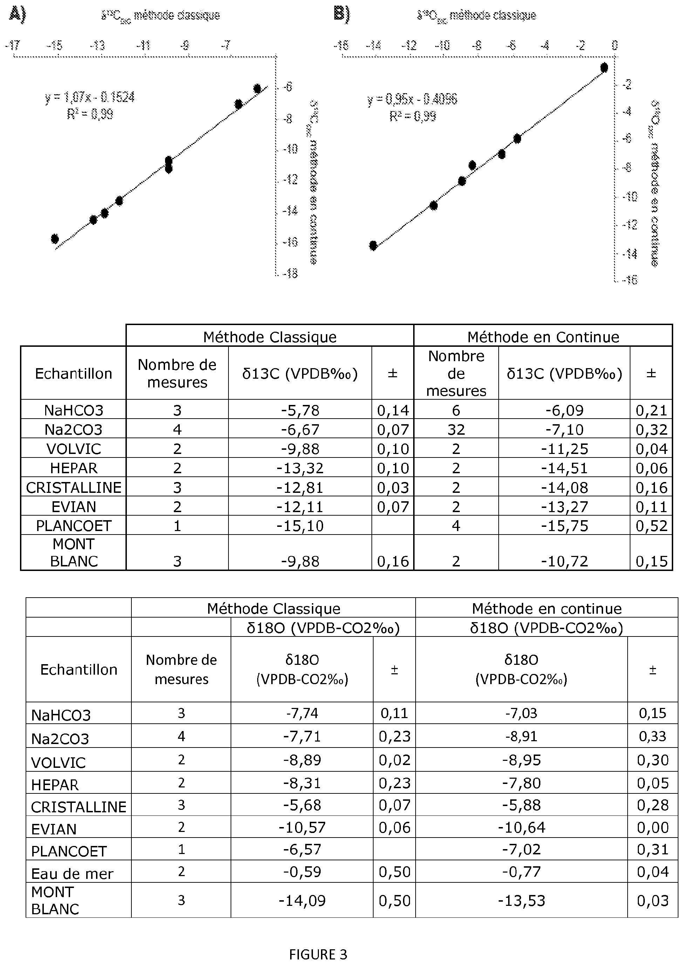

Les résultats sont présentés dans les figures 3A et 3B.
Les résultats pour chaque échantillon testé sont détaillés dans les tableaux en dessous des figures 3A et 3B.

**EXEMPLE 2 : EFFETS DE LA CONCENTRATION SUR LES VALEURS ISOTOPIQUES EN CARBONE ET EN OXYGÈNE DU DIC D'ÉCHANTILLONS LIQUIDES**

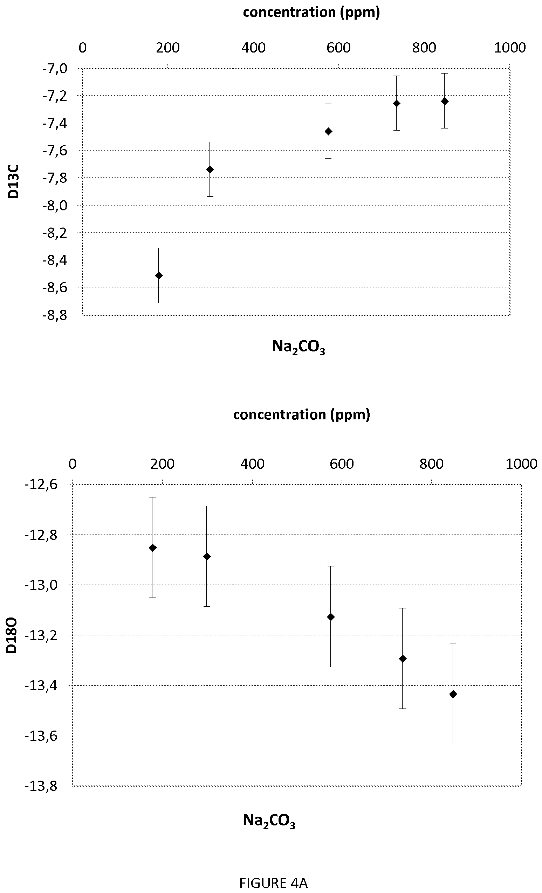

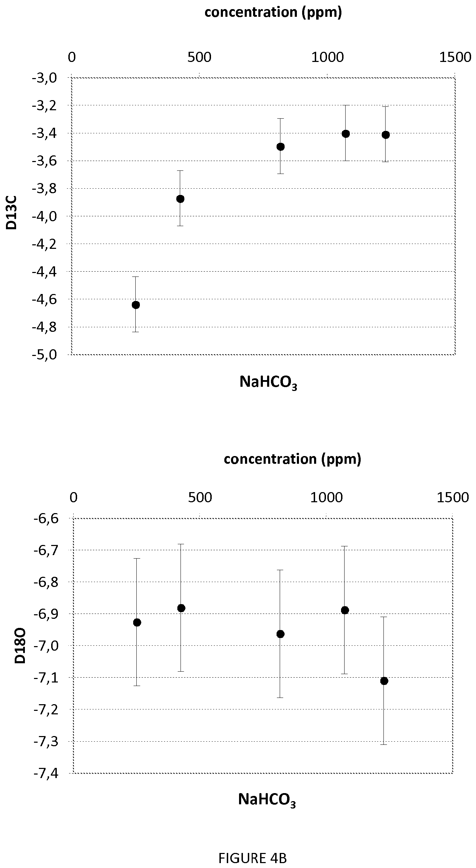

Pour cet exemple deux standards ($Na_2CO_3$ pour analyse Merck et NaHCOs pour analyse Merck) ont été préparés. Les standards ont été dissous dans de l'eau distillée afin d'obtenir 5 concentrations différentes : 150 ; 300 ; 600 ; 800 et 1000 ppm ; cela a permis de déterminer l'influence de la concentration de $CO_2$ dans la solution sur la mesure du $\delta^{13}C$ et du $\delta^{18}O$ avec le générateur d'hydrure utilisé selon la présente invention. Pour cette expérience la taille de la boucle a été fixée à 500 $\mu l$ et la vitesse de la pompe à 40 rpm.
Les résultats sont présentés dans la figure 4.

**EXEMPLE 3 : EFFETS DE LA VITESSE DE LA POMPE SUR LA CONCENTRATION ET LES VALEURS ISOTOPIQUES EN CARBONE ET EN OXYGÈNE DU DIC D'ÉCHANTILLONS LIQUIDES**

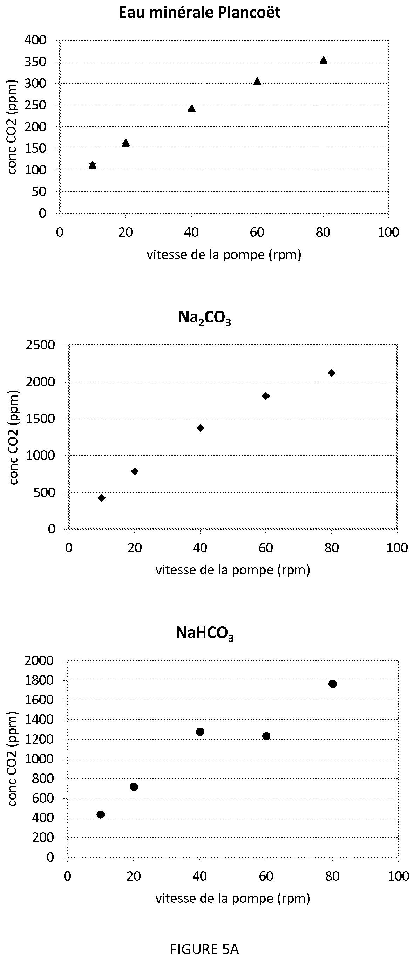

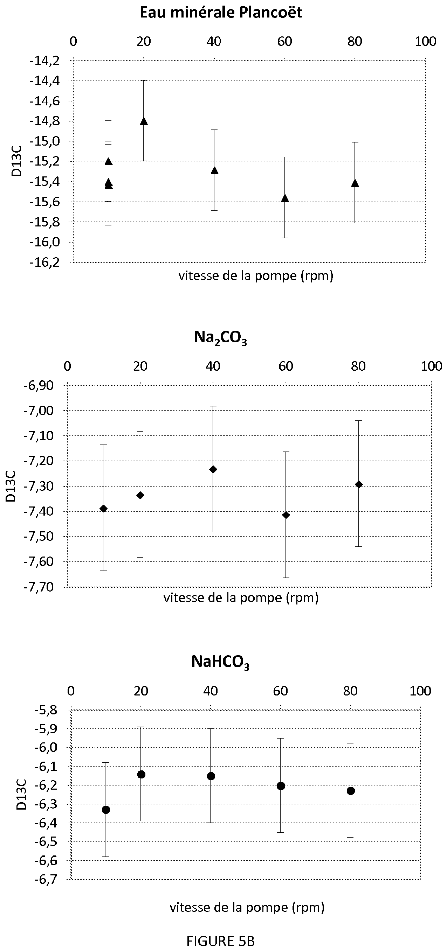

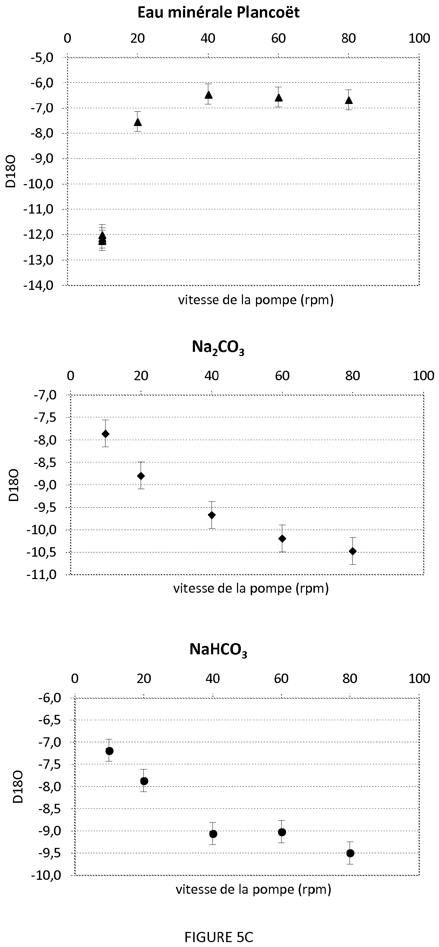

Pour cet exemple où le générateur d'hydrure a été utilisé selon la présente invention, 5 vitesses de pompe péristaltique ont été testées : 10 ; 20 ; 40 ; 60 et 80 rpm. Pour cette expérience la taille de la boucle a été fixée à 500 $\mu l$ et 3 échantillons ont été mesurés : une eau minérale Plancoët, $Na_2CO_3$ (1000 ppm) et NaHCOs (1000 ppm). Il apparaît que plus la vitesse de la pompe augmente plus la concentration en $CO_2$ est élevée, l'échange entre l'acide et l'échantillon semble plus efficace, par contre la valeur de $\delta^{13}C$ mesurée est relativement stable. Le $\delta^{18}O$, pour des vitesses de pompes faibles, montre une certaine variabilité, due probablement aux faibles concentrations en $CO_2$ produites.
Les résultats sont présentés dans la figure 5.

**EXEMPLE 4 : EFFETS DE LA LONGUEUR DE LA BOUCLE RÉACTIONNELLE SUR LES CONCENTRATIONS EN $CO_2$ ET LES VALEURS ISOTOPIQUES EN CARBONE ET EN OXYGÈNE DU DIC D'ÉCHANTILONS LIQUIDES**

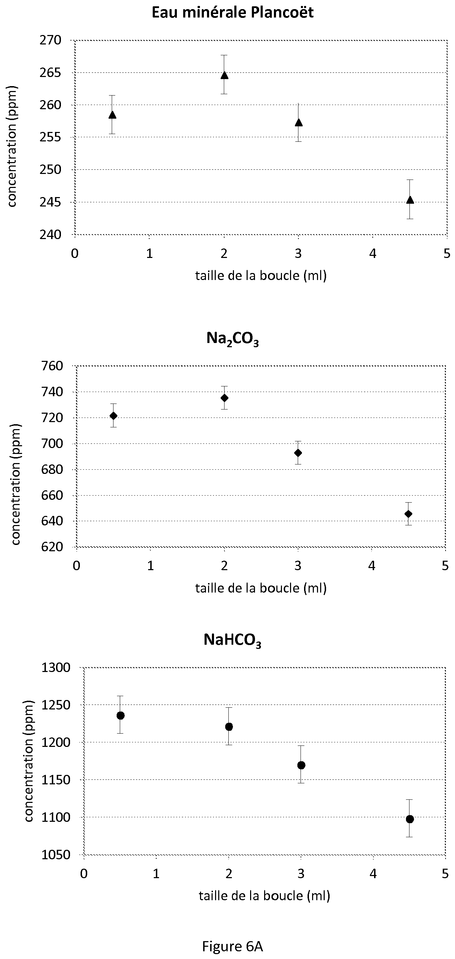

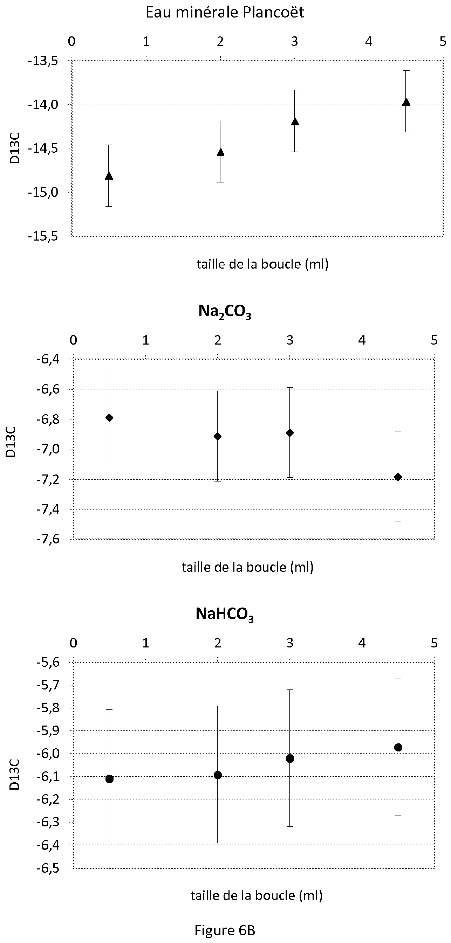

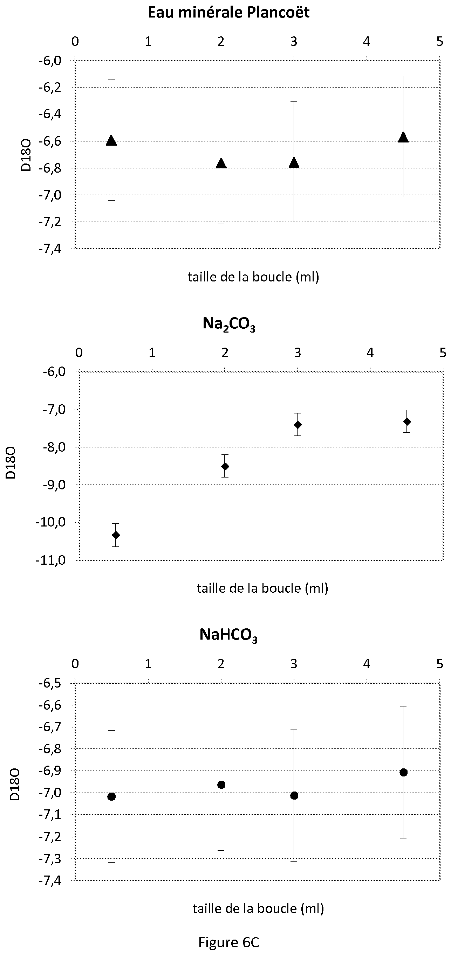

Pour cet exemple où le générateur d'hydrure a été utilisé selon la présente invention, 4 longueurs de boucles ont été testé : 0,5 ; 2 ; 3 et 4,5 ml. Pour cette expérience, la vitesse de la pompe a été fixée à 40 rpm et 3 échantillons ont été testés : une eau minérale Plancoët, $Na_2CO_3$ (1000 ppm) et NaHCOs (1000 ppm). Il apparaît que plus la taille de la boucle est grande plus la concentration en $CO_2$ diminue, l'échange entre l'acide et l'échantillon est possiblement moins efficace.
Les résultats sont présentés dans la figure 6.

**EXEMPLE 5 : CALCUL DU TEMPS DE RÉSIDENCE DANS DIFFÉRENTES CONDITIONS EXPÉRIMENTALES (VITESSE DE POMPE, VOLUME DE CHAMBRE, DÉBIT DE GAZ VECTEUR)**

Pour cet exemple, le générateur d'hydrure a également été utilisé selon la présente invention et les caractéristiques du tableau (a) ci-dessous.

(a) Configuration utilisée du séparateur gaz-liquide

| | |
|---|---|
| Diamètre interne des tubes pour les liquides (mm) | 0,3 |
| Débit de gaz (ml/s) (80ml/min pour Delta Ray) | 1,33 |
| Volume de la chambre (ml, estimation) | 8 |
| Vitesse de la pompe (rpm) | 40 |

Les résultats sont présentés dans les tableaux ci-dessous.

(b) Calcul du temps de résidence (TR) du $CO_2$ par la méthode continue (secondes)

$$TR\ total = volume\ total\ /\ (volume\ total\ \times\ secondes^{-1})$$

$$TR\ gaz = volume\ total\ /\ (volume\ de\ gaz\ \times\ secondes^{-1})$$

$$Volume\ total = volume\ de\ liquide + volume\ de\ gaz$$

(c) Calcul du temps de résidence (TR) pour différentes vitesses de pompe, en configuration typique

| TR total (s) | TR gaz | vitesse pompe (rpm) | débit de liquide ($\mu$l/s) | débit de gaz vecteur ($\mu$l/s) | débit total ($\mu$l/s) |
|---|---|---|---|---|---|
| 5,98 | 6,00 | 10 | 4,0 | 1333,3 | 1337,3 |
| 5,96 | 6,00 | 20 | 7,9 | 1333,3 | 1341,2 |
| 5,95 | 6,00 | 30 | 11,9 | 1333,3 | 1345,2 |
| 5,93 | 6,00 | 40 | 15,8 | 1333,3 | 1349,1 |
| 5,91 | 6,00 | 50 | 19,8 | 1333,3 | 1353,1 |
| 5,90 | 6,00 | 60 | 23,7 | 1333,3 | 1357,0 |
| 5,88 | 6,00 | 70 | 27,7 | 1333,3 | 1361,0 |

(d) Calcul du temps de résidence (TR) pour différents volumes de chambre, en configuration typique

| TR total (s) | TR gaz | volume chambre (ml) | débit de liquide ($\mu$l/s) | débit de gaz vecteur ($\mu$l/s) | débit total ($\mu$l/s) |
|---|---|---|---|---|---|
| 0,37 | 0,38 | 0,5 | 15,8 | 1333,3 | 1349,1 |
| 0,74 | 0,75 | 1 | 15,8 | 1333,3 | 1349,1 |
| 1,48 | 1,50 | 2 | 15,8 | 1333,3 | 1349,1 |
| 2,96 | 3,00 | 4 | 15,8 | 1333,3 | 1349,1 |
| 5,93 | 6,00 | 8 | 15,8 | 1333,3 | 1349,1 |
| 11,86 | 12,00 | 16 | 15,8 | 1333,3 | 1349,1 |

(suite)

| TR total (s) | TR gaz | volume chambre (ml) | débit de liquide ($\mu$l/s) | débit de gaz vecteur ($\mu$l/s) | débit total ($\mu$l/s) |
|---|---|---|---|---|---|
| 23,72 | 24,00 | 32 | 15,8 | 1333,3 | 1349,1 |

(e) Calcul du temps de résidence (TR) pour différents débits de gaz vecteur

| TR total (s) | TR gaz | débit de liquide ($\mu$l/s) | débit de gaz vecteur ($\mu$l/s) | débit total ($\mu$l/s) |
|---|---|---|---|---|
| 69,08 | 80,00 | 15,8 | 100,0 | 115,8 |
| 37,07 | 40,00 | 15,8 | 200,0 | 215,8 |
| 19,24 | 20,00 | 15,8 | 400,0 | 415,8 |
| 9,81 | 10,00 | 15,8 | 800,0 | 815,8 |
| 4,95 | 5,00 | 15,8 | 1600,0 | 1615,8 |
| 2,49 | 2,50 | 15,8 | 3200,0 | 3215,8 |
| 1,25 | 1,25 | 15,8 | 6400,0 | 6415,8 |

Les résultats montrent que le temps de résidence total moyen en utilisant ce type de montage est calculé à environ 6s. Il est possible de diminuer ce temps à 1s en diminuant le volume de la chambre de séparation.

**Liste des références**

1. Method 4500-CO2 dans Rice EW, Bridgewater L, Association APH, Association AWW, Fédération WE « Standard Methods for the Exalination of Water and Wastewater », Am. Public Health Assn., 2012
2. Assayag et al., Rapid Communications in Mass Spectrometry, 20 : 2243-2251, 2006
3. Bass et al., Rapid Communications in Mass Spectrometry, 26 : 639-644, 2012
4. Demande de Brevet US 2010/0212406
5. Using Isotope Ratio Infrared Spectrometer to détermine $\delta$13C and $\delta$18O of carbonate samples
6. Smajgl et al. 2017, 19th EGU General Assembly, EGU2017, proceedings from the conférence held 23-28 April, 2017 in Vienna, Austria., p.9793
7. Brevet US 8,114,676 B2

**Revendications**

1. Dispositif d'analyse en continu de la concentration du Carbone Inorganique Dissous (DIC) et de ses compositions isotopiques en carbone et en oxygène dans un échantillon liquide, ledit dispositif comprenant :

   - une pluralité de réservoirs, chacune comprenant un fluide ;
   - un assemblage de tubes chacun connecté à un réservoir (1),(2) contenant chacun un fluide, et connectés à une pompe (5) positionnée en aval desdits tubes ;
   - une chambre de mélange (6) positionnée en aval de la pompe (5), ladite chambre de mélange (6) permettant de mélanger dynamiquement les fluides acheminés via la pompe (5) ;
   - une boucle réactionnelle (7) positionnée en aval de la chambre de mélange (6), ladite boucle réactionnelle (7) permettant de convertir le DIC en $CO_2$ gazeux selon une réaction acide-base ;
   - une chambre de séparation (12) positionnée en aval de la boucle réactionnelle (7), ladite chambre de séparation (12) étant également connectée à une bouteille de gaz vecteur (8) contenant du gaz vecteur sans $CO_2$ ;
   - un détendeur (9) et un boitier de contrôle (10) positionnés entre la bouteille de gaz vecteur (8) et la chambre de séparation (12) et qui contrôlent le flux (11) de gaz vecteur envoyé de la bouteille de gaz vecteur (8) dans la chambre de séparation (12) pour capter le $CO_2$ produit dans la boucle réactionnelle (7) ;

- un réservoir de déchet (15) positionné en aval de la chambre de séparation (12) récupérant via la pompe (5) les fluides résiduels dégazés ;
- un piège à eau et à particules (18) positionné en aval de la chambre de séparation ;
- éventuellement un open-split (17) positionné entre la chambre de séparation (12) et le piège à eau et à particules (18) afin d'ajuster les flux ;
- un système de détection de la concentration du Carbone Inorganique Dissous et des ses compositions isotopiques en carbone et oxygène (19) positionné en aval du piège à eau et à particules (18).

2. Dispositif selon la revendication 1, dans lequel la chambre de réaction (12) comprend une paroi à surface étendue et traitée pour optimiser le dégazage (13).

3. Dispositif selon la revendication 2, dans lequel la paroi de la chambre de séparation (12) est en verre dépoli.

4. Dispositif selon l'une quelconque des revendications 1 3, dans lequel le réservoir (1) contient l'échantillon liquide à analyser, et le réservoir (2) contient un réactif acidifiant, de préférence un acide anhydrique, tout préférentiellement un acide orthophosphorique.

5. Dispositif selon l'une quelconque des revendications 1 à 4, dans lequel le système de détection (19) est un analyseur de type spectromètre infra-rouge à ratio isotopique.

6. Dispositif selon l'une quelconque des revendications 1 à 5, dans lequel le gaz vecteur est choisi parmi l'hélium et de l'air synthétique sans $CO_2$.

7. Méthode d'analyse en continu de la concentration du Carbone Inorganique Dissous (DIC) et de ses compositions isotopiques en carbone et en oxygène dans un échantillon liquide, ledit procédé comprenant :

a) l'acheminement de l'échantillon liquide à analyser et d'un réactif acidifiant vers une pompe (5) avec des flux constants F1 (3) et F2 (4), respectivement ;
b) l'injection et le mélange des deux fluides dans une chambre de mélange (6) ;
c) l'acheminement du mélange de fluides obtenu à l'étape b) vers une boucle réactionnelle (7) dans laquelle une réaction acide-base permet de convertir en tout ou partie le DIC en $CO_2$ gazeux ;
d) l'acheminement du mélange de gaz et de liquide obtenu à l'étape c) dans une chambre de séparation (12) ;
e) l'injection d'un flux (11) de gaz vecteur dépourvu de $CO_2$ dans la chambre de séparation (12) afin de capter le $CO_2$ gazeux produit lors de l'étape c) et libéré (13) dans la chambre de séparation (12) ;
f) l'acheminement du flux (16) de gaz vecteur contenant le $CO_2$ dégazé de l'échantillon obtenu à l'étape e) à travers un piège à eau et à particules (18), éventuellement après un passage dans un open-split (17) pour un ajustement de flux ;
g) l'acheminement du fluide résiduel composé du réactif acidifiant et de l'échantillon dégazé dans un réservoir de déchets (15) ;
h) l'acheminement du flux (16) de gaz vecteur contenant le $CO_2$ gazeux issu du DIC dans un système de détection (19) ;
i) la détermination de la concentration du DIC et de ses compositions isotopiques en carbone et en oxygène par ledit système de détection (19).

8. Procédé selon la revendication 7, où l'étape a) est réalisée sous des flux constants F1 (3) d'échantillon et F2 (4) de réactif acidifiant de l'ordre de 750 $\mu$l/min et 190 $\mu$l/min, respectivement.

9. Procédé selon l'une quelconque des revendications 7 à 8, où l'étape e) est réalisée avec une boucle réactionnelle dont la longueur varie de 0,5 à 4,5 ml en fonction de l'échantillon à analyser.

10. Procédé selon l'une quelconque des revendications 7 à 9, où l'étape e) utilise un gaz vecteur choisi parmi l'hélium et l'air synthétique sans $CO_2$.

11. Procédé selon l'une quelconque des revendications 7 à 10, où le flux (11) est contrôlé par un détendeur (9) et un boîtier de contrôle (10) afin d'optimiser la quantité de $CO_2$ gazeux arrivant au système de détection (19).

12. Procédé selon l'une quelconque des revendications 7 à 11, où l'étape a) est réalisée avec un réactif acidifiant qui est un acide anhydrique, de préférence un acide orthophosphorique.

13. Procédé selon l'une quelconque des revendications 7 à 12, où l'étape i) est réalisée à l'aide d'un analyseur de type spectromètre laser-infrarouge à ratio isotopique.

**Patentansprüche**

1. Vorrichtung zur kontinuierlichen Analyse der Konzentration von gelöstem anorganischem Kohlenstoff (DIC) und seiner isotopischen Kohlenstoff- und Sauerstoffzusammensetzung in einer flüssigen Probe, wobei die Vorrichtung umfasst:

   - eine Vielzahl von Behältern, die jeweils eine Flüssigkeit enthalten;
   - eine Anordnung von Schläuchen, die jeweils mit einem Behälter (1), (2) verbunden sind, die jeweils ein Fluid enthalten, und die mit einer Pumpe (5) verbunden sind, die stromabwärts von den Schläuchen angeordnet ist;
   - eine Mischkammer (6), die stromabwärts der Pumpe (5) angeordnet ist, wobei die Mischkammer (6) es ermöglicht, die durch die Pumpe (5) geförderten Fluide dynamisch zu mischen;
   - eine Reaktionsschleife (7), die stromabwärts der Mischkammer (6) angeordnet ist, wobei die Reaktionsschleife (7) die Umwandlung des DIC in $CO_2$ Gas gemäß einer Säure-Base-Reaktion ermöglicht;
   - eine Trennkammer (12), die stromabwärts der Reaktionsschleife (7) angeordnet ist, wobei die Trennkammer (12) auch mit einem Vektorgaszylinder (8) verbunden ist, der $CO_2$-freies Vektorgas enthält;
   - einen Druckregler (9) und eine Steuereinheit (10), die zwischen dem Vektorgaszylinder (8) und der Abtrennkammer (12) angeordnet sind und die den Strom (11) des vom Vektorgaszylinder (8) in die Abtrennkammer (12) geleiteten Vektorgases zum Auffangen des in der Reaktionsschleife (7) erzeugten $CO_2$ steuern;
   - einem stromabwärts der Trennkammer (12) angeordneten Abfallbehälter (15) zur Rückgewinnung der entgasten Restflüssigkeiten über die Pumpe (5);
   - eine Wasser- und Partikelfalle (18), die stromabwärts der Trennkammer angeordnet ist;
   - optional einen offenen Spalt (17), der zwischen der Trennkammer (12) und der Wasser- und Partikelfalle (18) angeordnet ist, um die Durchflüsse zu regulieren;
   - ein System (19) zum Nachweis der Konzentration von gelöstem anorganischem Kohlenstoff (DIC) und seiner isotopischen Kohlenstoff- und Sauerstoffzusammensetzung, das stromabwärts der Wasser- und Partikelfalle (18) angeordnet ist.

2. Vorrichtung nach Anspruch 1, wobei die Reaktionskammer (12) eine Wand mit einer erweiterten und behandelten Oberfläche zur Optimierung der Entgasung (13) aufweist.

3. Vorrichtung nach Anspruch 2, wobei die Wand der Trennkammer (12) aus mattiertem Glas besteht.

4. Vorrichtung nach einem der Ansprüche 1 bis 3, **dadurch gekennzeichnet, dass** der Vorratsbehälter (1) die zu analysierende flüssige Probe und der Vorratsbehälter (2) ein Ansäuerungsreagenz, vorzugsweise eine anhydrische Säure, besonders bevorzugt eine Orthophosphorsäure, enthält.

5. Vorrichtung nach einem der Ansprüche 1 bis 4, wobei das Detektionssystem (19) ein Analysator des Typen Isotopenverhältnis-Infrarotspektrometer ist.

6. Vorrichtung nach einem der Ansprüche 1 bis 5, wobei das Vektorgas aus Helium und $CO_2$-freier synthetischer Luft ausgewählt wird.

7. Verfahren zur kontinuierlichen Analyse der Konzentration von gelöstem anorganischem Kohlenstoff (DIC) und seiner isotopischen Kohlenstoff- und Sauerstoffzusammensetzung in einer flüssigen Probe, wobei das Verfahren umfasst:

   a) Förderung der zu analysierende flüssige Probe und eines Säurereagenzes zu einer Pumpe (5) mit konstantem Durchfluss F1 (3) bzw. F2 (4);
   b) Einspritzen und Mischen der beiden Flüssigkeiten in einer Mischkammer (6);
   c) das in Schritt b) erhaltene Flüssigkeitsgemisch in eine Reaktionsschleife (7) zu leiten, in der eine Säure-Base-Reaktion die vollständige oder teilweise Umwandlung des DIC in $CO_2$ Gas ermöglicht;
   d) Fördern des in Schritt c) erhaltenen Gas-Flüssigkeits-Gemisches in eine Trennkammer (12);
   e) Einleiten eines Stroms (11) von CO-freiem Vektorgas$_2$ in die Trennkammer (12), um das in Schritt c) erzeugte und in der Trennkammer (12) freigesetzte $CO_2$ einzufangen (13);
   f) Leiten des in Schritt e) erhaltenen Stroms (16) des Vektorgases, das das aus der Probe entgaste $CO_2$ enthält,

durch eine Wasser- und Partikelfalle (18), gegebenenfalls nach einem Durchgang durch einen offenen Spalt (17) für eine Durchflusseinstellung;

g) Fördern der Restflüssigkeit, die sich aus dem Säurereagenz und der entgasten Probe zusammensetzt, in einen Abfallbehälter (15);

h) Weiterleitung des Gasstroms (16), der das aus dem DIC abgeleitete $CO_2$ enthält, in ein Detektionssystem (19);

i) Bestimmung der Konzentration des DIC und/oder seiner isotopischen Kohlenstoff- und Sauerstoffzusammensetzung mit Hilfe des Detektionssystems (19).

**8.** Verfahren nach Anspruch 7, wobei Schritt a) unter konstanten Flüssen F1 (3) der Probe und F2 (4) des Ansäuerungsreagenzes in der Größenordnung von 750 µl/min bzw. 190 µl/min durchgeführt wird.

**9.** Verfahren nach einem der Ansprüche 7 oder 8, wobei Schritt e) mit einer Reaktionsschleife durchgeführt wird, deren Länge in Abhängigkeit von der zu analysierende Probe zwischen 0,5 und 4,5 ml liegt.

**10.** Verfahren nach einem der Ansprüche 7 bis 9, wobei in Schritt e) ein Vektorgas verwendet wird, das aus Helium und $CO_2$ -freier synthetischer Luft ausgewählt wird.

**11.** Verfahren nach einem der Ansprüche 7 bis 10, wobei der Durchfluss (11) durch einen Druckregler (9) und eine Steuereinheit (10) gesteuert wird, um die Menge des am Detektionssystem (19) ankommenden $CO_2$ Gases zu optimieren.

**12.** Verfahren nach einem der Ansprüche 7 bis 11, wobei Schritt a) mit einem Ansäuerungsreagenz durchgeführt wird, dass eine wasserfreie Säure, vorzugsweise eine Orthophosphorsäure ist.

**13.** Verfahren nach einem der Ansprüche 7 bis 12, wobei Schritt i) mit einem Analysator vom Typ Isotopenverhältnis-Infrarot-Laserspektrometer durchgeführt wird.

**Claims**

**1.** A device for continuous analysis of the concentration of dissolved inorganic carbon (DIC) and of the isotopic carbon and oxygen compositions thereof in a liquid sample, said device comprising:

- a plurality of reservoirs, each comprising a fluid;
- an assembly of tubes, each connected to a reservoir (1),(2), each containing a fluid, and connected to a pump (5) positioned downstream of said tubes;
- a mixing chamber (6) positioned downstream of the pump (5), said mixing chamber (6) making it possible to dynamically mix the fluids conveyed via the pump (5);
- a reaction loop (7) positioned downstream of the mixing chamber (6), said reaction loop (7) making it possible to convert the DIC into $CO_2$ gas according to an acid-base reaction;
- a separation chamber (12) positioned downstream of the reaction loop (7), said separation chamber (12) also being connected to a vector-gas cylinder (8) containing $CO_2$-free vector gas;
- a pressure regulator (9) and a control unit (10) positioned between the vector-gas cylinder (8) and the separation chamber (12) and which control the flow (11) of vector gas sent from the vector-gas cylinder (8) into the separation chamber (12) for capturing the $CO_2$ produced in the reaction loop (7);
- a waste reservoir (15) positioned downstream of the separation chamber (12) for recovering, via the pump (5), the degassed residual fluids;
- a water- and particle-trap (18) positioned downstream of the separation chamber;
- optionally, an open-split (17) positioned between the separation chamber (12) and the water- and particle-trap (18) in order to adjust the flows;
- a detection system (19) of the concentration of dissolved inorganic carbon (DIC) and of the isotopic carbon and oxygen compositions thereof, positioned downstream of the water- and particle-trap (18).

**2.** The device as claimed in claim 1, wherein the reaction chamber (12) comprises a wall with an extended and treated surface for optimizing the degassing (13).

**3.** The device as claimed in claim 2, wherein the wall of the separation chamber (12) is made of frosted glass.

4. The device as claimed in any one of claims 1 to 3, wherein the reservoir (1) contains the liquid sample to be analyzed, and the reservoir (2) contains an acidifying reagent, preferably an anhydric acid, most preferentially an orthophosphoric acid.

5. The device as claimed in any one of claims 1 to 4, wherein the detection system (19) is an analyzer of isotope-ratio infrared spectrometer type.

6. The device as claimed in any one of claims 1 to 5, wherein the vector gas is chosen from helium and CO2-free synthetic air.

7. A method for continuous analysis of the concentration of dissolved inorganic carbon (DIC) and of the isotopic carbon and oxygen compositions thereof in a liquid sample, said method comprising:

   a) conveying the liquid sample to be analyzed and an acidifying reagent to a pump (5) with constant flows F1 (3) and F2 (4), respectively;
   b) injecting and mixing the two fluids in a mixing chamber (6);
   c) conveying the mixture of fluids obtained in step b) to a reaction loop (7) wherein an acid-base reaction makes it possible to totally or partially convert the DIC into $CO_2$ gas;
   d) conveying the mixture of gas and liquid obtained in step c) into a separation chamber (12);
   e) injecting a flow (11) of vector gas devoid of $CO_2$ into the separation chamber (12) in order to capture the $CO_2$ gas produced during step c) and released (13) in the separation chamber (12);
   f) conveying the flow (16) of vector gas containing the $CO_2$ degassed from the sample, obtained in step e), through a water- and particle-trap (18), optionally after a passage through an open-split (17) for a flow adjustment;
   g) conveying the residual fluid composed of the acidifying reagent and of the degassed sample into a waste reservoir (15);
   h) conveying the flow (16) of vector gas containing the $CO_2$ gas derived from the DIC into a detection system (19);
   i) determining the concentration of the DIC and/or the isotopic carbon and oxygen compositions thereof by means of said detection system (19).

8. The method as claimed in claim 7, wherein step a) is carried out under constant flows F1 (3) of sample and F2 (4) of acidifying reagent of the order of 750 $\mu$l/min and 190 $\mu$l/min, respectively.

9. The method as claimed in either one of claims 7 and 8, wherein step e) is carried out with a reaction loop of which the length ranges from 0.5 to 4.5 ml as a function of the sample to be analyzed.

10. The method as claimed in any one of claims 7 to 9, wherein step e) uses a vector gas chosen from helium and $CO_2$-free synthetic air.

11. The method as claimed in any one of claims 7 to 10, wherein the flow (11) is controlled by a pressure regulator (9) and a control unit (10) in order to optimize the amount of $CO_2$ gas arriving at the detection system (19).

12. The method as claimed in any one of claims 7 to 11, wherein step a) is carried out with an acidifying reagent which is an anhydric acid, preferably an orthophosphoric acid.

13. The method as claimed in any one of claims 7 to 12, wherein step i) is carried out by means of an analyzer of isotope-ratio infrared laser spectrometer type.

FIGURE 1

FIGURE 2

A) δ¹³C_DIC méthode classique

$$y = 1,07x - 0,1524$$
$$R^2 = 0,99$$

B) δ¹⁸O_DIC méthode classique

$$y = 0,95x - 0,4096$$
$$R^2 = 0,99$$

| | Méthode Classique | | | Méthode en Continue | | |
|---|---|---|---|---|---|---|
| Echantillon | Nombre de mesures | δ13C (VPDB‰) | ± | Nombre de mesures | δ13C (VPDB‰) | ± |
| NaHCO3 | 3 | -5,78 | 0,14 | 6 | -6,09 | 0,21 |
| Na2CO3 | 4 | -6,67 | 0,07 | 32 | -7,10 | 0,32 |
| VOLVIC | 2 | -9,88 | 0,10 | 2 | -11,25 | 0,04 |
| HEPAR | 2 | -13,32 | 0,10 | 2 | -14,51 | 0,06 |
| CRISTALLINE | 3 | -12,81 | 0,03 | 2 | -14,08 | 0,16 |
| EVIAN | 2 | -12,11 | 0,07 | 2 | -13,27 | 0,11 |
| PLANCOET | 1 | -15,10 | | 4 | -15,75 | 0,52 |
| MONT BLANC | 3 | -9,88 | 0,16 | 2 | -10,72 | 0,15 |

| | Méthode Classique | | | Méthode en continue | | |
|---|---|---|---|---|---|---|
| | δ18O (VPDB-CO2‰) | | | δ18O (VPDB-CO2‰) | | |
| Echantillon | Nombre de mesures | δ18O (VPDB-CO2‰) | ± | δ18O (VPDB-CO2‰) | ± | |
| NaHCO3 | 3 | -7,74 | 0,11 | -7,03 | 0,15 | |
| Na2CO3 | 4 | -7,71 | 0,23 | -8,91 | 0,33 | |
| VOLVIC | 2 | -8,89 | 0,02 | -8,95 | 0,30 | |
| HEPAR | 2 | -8,31 | 0,23 | -7,80 | 0,05 | |
| CRISTALLINE | 3 | -5,68 | 0,07 | -5,88 | 0,28 | |
| EVIAN | 2 | -10,57 | 0,06 | -10,64 | 0,00 | |
| PLANCOET | 1 | -6,57 | | -7,02 | 0,31 | |
| Eau de mer | 2 | -0,59 | 0,50 | -0,77 | 0,04 | |
| MONT BLANC | 3 | -14,09 | 0,50 | -13,53 | 0,03 | |

FIGURE 3

concentration (ppm)

Na$_2$CO$_3$

concentration (ppm)

Na$_2$CO$_3$

FIGURE 4A

FIGURE 4B

## Eau minérale Plancoët

## $Na_2CO_3$

## $NaHCO_3$

FIGURE 5A

## Eau minérale Plancoët

## Na$_2$CO$_3$

## NaHCO$_3$

FIGURE 5B

## Eau minérale Plancoët

## Na$_2$CO$_3$

## NaHCO$_3$

FIGURE 5C

## Eau minérale Plancoët

## Na$_2$CO$_3$

## NaHCO$_3$

Figure 6A

Eau minérale Plancoët

$Na_2CO_3$

$NaHCO_3$

Figure 6B

**Eau minérale Plancoët**

**Na$_2$CO$_3$**

**NaHCO$_3$**

Figure 6C

concentration en $CO_2$ à 40 rpm
lecture sur la bouteille/
méthode en continue

$R^2 = 0,9976$

|  | méthode continue | lecture sur la bouteille |
|---|---|---|
| Volvic | 220 | 74 |
| Evian | 834 | 360 |
| Cristalline ELEONORE | 597 | 236 |
| Contrex | 855 | 372 |
| Vittel | 859 | 384 |
| Mont Blanc | 208 | 65 |
| Plancoët | 325 | 121 |

FIGURE 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20100212406 A
- US 8114676 B
- US 8114676 B2

**Littérature non-brevet citée dans la description**

- **RICE EW ; BRIDGEWATER L ; ASSOCIATION APH ; ASSOCIATION AWW ; FÉDÉRATION WE.** Standard Methods for the Examination of Water and Wastewater. *Am. Public Health Assn.,* 2012
- **SMAJGL et al.** *19th EGU General Assembly, EGU2017, proceedings from the conférence,* 2017, 9793
- **RICE EW ; BRIDGEWATER L ; ASSOCIATION APH ; ASSOCIATION AWW ; FÉDÉRATION WE.** Standard Methods for the Exalination of Water and Wastewater. *Am. Public Health Assn.,* 2012
- **ASSAYAG et al.** *Rapid Communications in Mass Spectrometry,* 2006, vol. 20, 2243-2251
- **BASS et al.** *Rapid Communications in Mass Spectrometry,* 2012, vol. 26, 639-644
- **SMAJGL et al.** *19th EGU General Assembly, EGU2017, proceedings from the conférence,* 23 Avril 2017